# DEMANDE DE BREVET EUROPEEN

(11) **EP 0 627 446 A1**
(43) Date de publication de la demande: **07.12.1994**
(21) Numéro de dépôt: 94401187.3
(22) Date de dépôt: 30.05.1994
(51) Int. Cl.: C08B 37/16, A61K 47/48

(54) **Dérivés sulfones de cyclomalto-oligosaccharides, leur procédé de préparation et support de substances actives contenant ces dérivés**

(30) Priorité: 03.06.1993 FR 9306654
(71) Demandeur: COMMISSARIAT A L'ENERGIE ATOMIQUE, F-75015 Paris (FR)
(72) Inventeur: Gadelle, Andrée, F-38330 Montbonnot (FR); Bayle, Corine, F-63530 Volvic (FR)
(74) Mandataire: Dubois-Chabert, Guy

(57) **Abrégé**

Dérivés sulfonés de cyclomalto-oligosaccharides, leur procédé de préparation et support de substances actives contenant ces dérivés.

Ces dérivés présentent la formule (I) suivante : 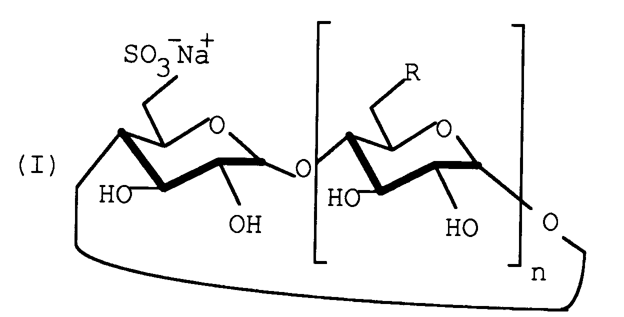
dans laquelle n est un entier allant de 2 à 50 et R représente -SO₃⁻Na⁺ ou -OH, R pouvant être différent d'un cycle à l'autre. Ils peuvent être utilisés pour le transport de médicaments injectables ou ingérables.

## Description

La présente invention a pour objet de nouveaux dérivés de cyclomalto-oligosaccharides, leur procédé de préparation ainsi qu'un support de substances actives contenant ces dérivés.

De façon plus précise, l'invention concerne des dérivés cyclomalto-oligosaccharides anioniques obtenus en fixant sur au moins un des carbones en position 6 de l'oligosaccharide cyclique un groupement sulfonate.

Les cyclomalto-oligosaccharides, plus couramment appelés cyclodextrines, sont des oligosaccharides cycliques du D-glucose comportant le plus communément de six à huit unités D-glucopyranosyle liées par des enchaînements α-(1→4), ce qui leur confère une morphologie de type torique. Du fait de cette géométrie moléculaire et des structures électroniques afférentes, un caractère hydrophobe est associé à la cavité interne du cycle alors que la surface externe de la molécule est hydrophile.

Cette disposition favorise la formation de complexes d'inclusion avec des molécules hydrophobes par association apolaire, améliorant ainsi la solubilisation de ces molécules dans l'eau. Ainsi, les cyclodextrines sont-elles utilisées pour stabiliser des molécules labiles, améliorer le transport moléculaire de substances ou principes actifs vers des sites cibles, ou encore favoriser des réactions d'interface comme décrit dans le document (1) Drug Development and Industrial Pharmacy, 12, 11-13, p. 2193-2216, 1986.

Ces propriétés sont très largement exploitées dans de nombreux domaines tels que les domaines pharmaceutique, vétérinaire, chimique, agrochimique, alimentaire et cosmétique incluant les arômes et parfums.

On sait que la solubilité des cyclodextrines dans l'eau et par voie de conséquence celle de leurs produits d'inclusion sont notablement augmentées lorsque la molécule est substituée par des groupements hydrophiles. C'est en particulier le cas des cyclodextrines comportant des groupements polaires chargés (aminés ou sulfatés).

Par ailleurs, les cyclodextrines à groupement sulfate présentent une activité hémolytique plus faible que celle de leurs homologues non sulfatés comme décrit dans le document (2), Biochemical Pharmacology, vol. 42, n°7, pp. 1502-1503, 1991, "Sulfation and hemolytic activity of cyclodextrin" de E.J. Macarak.

Ainsi, les nouveaux dérivés de cyclomalto-oligosaccharides de l'invention peuvent être utilisés dans toutes les applications connues des cyclodextrines et notamment comme encapsulant et/ou agent de transport de substances actives insolubles dans l'eau comme l'AZT (C & EN Rudy Baum and R. Dagami, 16/07/1990, p. 7-15) pour le traitement du SIDA.

En outre, ils peuvent être utilisés comme complexant de cations par exemple pour l'extraction d'ions uranyle (UO₂²⁺) en remplacement des calixarènes sulfonés comme décrit dans le document (3) S. Shinkai et al., J. Am. Chem. Soc. 1987, 109, p. 6371-6376, "Molecular design of calixarene-based uranophiles which exhibit remarkably high stability and selectivity". Ainsi, les dérivés de l'invention peuvent aussi être utilisés dans le domaine nucléaire pour la décontamination des effluents liquides.

Des cyclodextrines à propriétés anioniques sont en outre décrites dans le document (4) US-A-3 426 011. Les groupements anioniques de ces cyclodextrines sont en particulier des ethers sulfopropyliques ou sulfoéthyliques. Ces cyclodextrines à propriétés anioniques sont obtenues par action d'un halogénoalkyle sulfonate sur une cyclodextrine activée sous forme d'alcoolate. Cette réaction permet l'obtention de produits statistiquement substitués.

Des dérivés sulfonés de monosaccharides linéaires sont connus depuis longtemps, notamment du document (5) Carbohydrate Research, 22 (1972), p. 23-35 de J. Lehmann et W. Weckerle, "Zuckersulfonsäuren" ; du document (6) de M. Myano et A. Benson, J. Am. Chem. Soc., 84, 59 (1972), p. 57-62, "The plant sulfolipid. VI. Configuration of the glycerol moiety" ; du document (7) R. Whistler et D. Medcalf, Archives of Biochemistry and Biophysics 105, 1964, p. 1-6, "Preparation of 6-deoxyamylose-6-sulfonic acid".

Les procédés de fabrication de ces dérivés sulfonés sont généralement longs et complexes.

L'invention a pour objet de nouveaux dérivés de cyclomalto-oligosaccharides à propriétés anioniques, comportant au moins un groupement sulfonate, présentant toutes les propriétés et avantages des cyclodextrines à groupements sulfates, connues. En outre, leur procédé de fabrication est simple.

Selon une caractéristique principale de l'invention, les dérivés sulfonés de cyclomalto-oligosaccharides présentent la formule (I) suivante : 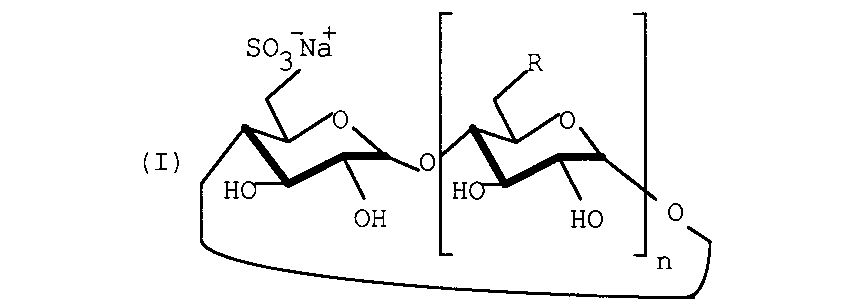
dans laquelle n est un entier allant de 2 à 50 et R représente -SO₃⁻Na⁺ ou -OH, R pouvant être différent d'un cycle à l'autre.

En particulier, n est choisi de 2 à 10.

Les groupements anioniques peuvent être agencés de façon symétrique ou non sur l'ensemble de l'oligosaccharide selon leur application.

Les dérivés sulfonés cycliques de l'invention présentent du fait de la présence d'un ou plusieurs groupements sulfonate une solubilité accrue dans l'eau par rapport à leurs homologues non sulfonés. Ces groupements confèrent en outre une grande stabilité aux dérivés et permettent leur orientation dans un champ électrique.

Ainsi, ils peuvent être utilisés en tant que supports de substances ou de principes actifs et en particulier en tant qu'encapsulant afin d'assurer le transport de ces substances ou principes actifs dans l'eau, où ils sont généralement insolubles.

En outre, les cyclomalto-oligosaccharides de l'invention ayant l'avantage, par rapport aux β-cyclodextrines naturelles d'être non hémolytiques, peuvent être utilisés pour la constitution de médicaments injectables par voie intraveineuse ou ingérables.

Aussi, l'invention a encore pour objet un support de substances actives contenant un dérivé de formule (I).

L'invention a aussi pour objet un procédé de préparation original de ces dérivés sulfonés. Selon une caractéristique principale de ce procédé, on fait réagir du sulfite de sodium sur un halogénure d'un cyclomalto-oligosaccharide correspondant, en présence d'un agent de transfert de phase, cet halogénure comportant au moins un atome d'halogène en position 6 d'un cycle glucosique.

Dans ce procédé, on utilise les propriétés de la mycellisation directe.

Les 6-halodérivés sont obtenus selon les procédés connus. En outre, la réaction d'échange d'un halogénure pour un sulfonate est connue depuis longtemps en chimie organique.

L'halogénure peut être un iodure, un bromure ou un chlorure. Il peut être monohalogéné, partiellement halogéné ou perhalogéné. La place des halogènes dans le cyclomalto-oligosaccharide de départ fixe la position des groupements sulfonate.

De façon avantageuse, la réaction est effectuée en milieu aqueux, l'agent de transfert de phase assurant la mise en solution de l'halogénure du cyclomalto-oligosaccharide normalement insoluble dans l'eau.

L'emploi d'un ou plusieurs solvants organiques tels que le diméthylsulfoxide (DMSO), le diméthylfluorure (DMF), la N-méthylpyrolidone additionnés éventuellement d'eau ne permet pas d'obtenir le produit recherché ; seul le produit de départ ou le dérivé 3-6-anhydro-D-glucose se retrouve dans le milieu réactionnel.

Un agent de transfert de phase, appelé aussi agent tensioactif, ou surfactant est un composé qui possède une tête polaire et une chaîne hydrophobe suffisamment longue entraînant des tendances respectivement hydrophiles et lipophiles fortement marquées. La dissolution d'une faible quantité de tensioactifs dans un solvant, polaire ou non, se manifeste par une forte diminution de la tension superficielle. De plus, au-delà de certains seuils de concentration caractéristiques, les solutions de tensioactifs sont capables d'incorporer des quantités importantes de composés normalement insolubles dans le milieu considéré.

Afin de favoriser l'approche de l'anion sulfite solubilisé dans l'eau vers les halogéno-sulfodextrines, on utilise de préférence un tensioactif à tête polaire chargée positivement par un groupement ammonium quaternaire ; la partie hydrophobe quant à elle, comporte des groupements alkyle à chaîne ayant de 2 à 15 atomes de carbone.

Le choix du contre-ion (ou anion) du tensioactif est important car il ne doit pas être compétiteur de l'anion sulfite dans la réaction d'échange halogénure-sulfite.

Comme contre-ion possible du tensioacif, on peut citer l'anion sulfate, sulfite, acétate, chlorure, bromure, iodure.

Ainsi, comme agent de transfert de masse utilisable dans l'invention, on peut citer l'hexadécyl triméthylammonium bromure ou le sulfate de méthyltrioctylammonium.

Afin d'éviter d'une part l'oxydation de l'ion sulfite en sulfate en milieu acide et par conséquent de provoquer la formation d'unités 6-désoxy-D-glucose sur la cyclodextrine halogénée de départ et d'autre part la formation d'unités 3,6-anhydro-D-glucose en milieu alcalin, la réaction d'échange se déroule de préférence en milieu neutre ou faiblement acide, c'est-à-dire à un pH allant de 4 à 8.

Le procédé de l'invention a l'avantage d'être plus simple que les procédés connus pour préparer des dérivés sulfonés de monosaccharide tels que celui décrit dans le document (7) pour la fabrication d'amylose ou de malto-oligosaccharide linéaire perhalogéné.

L'acide utilisé peut être organique ou minéral. Ainsi, le milieu réactionnel peut être un milieu sulfurique, sulfureux, chlorhydrique, iodhydrique, bromhydrique, acétique.

D'autres caractéristiques et avantages de l'invention ressortiront mieux à la lecture des exemples suivants de préparation de dérivés sulfonés donnés à titre illustratif et non limitatif.

### EXEMPLE PRELIMINAIRE : Préparation de la solution de transfert de phase.

44,2g (soit 0,1mol/l) de chlorure de méthyltrioctylammonium sont dissous dans 500ml d'eau et traités par 60g de résine basique du type Dowex^{R} SBR OH⁻, pendant 0,25 h. La résine est alors enlevée par filtration, soigneusement rincée avec de l'eau distillée. Les filtrats réunis sont neutralisés par une solution d'acide sulfurique (2N). Cette neutralisation est suivie par potentiométrie. Le volume de l'émulsion obtenue est complété à 1,51 et son pH ajusté à 6,5 par addition d'une solution de soude (1N). Cette émulsion stable d'agent tensioactif est utilisée directement dans les modes opératoires décrits ci-dessous de préparation de sel de sodium de cyclomalto-oligosaccharide sulfoné.

### EXEMPLE 1 : Synthèse du sel de sodium de l'heptakis(6-désoxy-6-sulfonyl)cyclomalto-heptaose.

A 120ml de l'émulsion d'agent tensioactif ci-dessus, sont ajoutés 900mg (7,14mmol) de sulfite de sodium et 900mg (3,31 méquiv.) d'heptakis (6-désoxy-6-iodo) cyclomalto-heptaose préparés selon le mode opératoire décrit par A. Gadelle et J. Defaye dans le document (8) Angew. Chem. Int. Ed. Engl. 30, 78-80 (1991), "Selective halogenation at primary positions of cyclomalto-oligosaccharides and a synthesis of per-3,6-anhydro-cyclomalto-oligosaccharides".

Le mélange est porté à 100°C pendant 24 h. Après refroidissement, le mélange réactionnel est extrait par du dichlorométhane en deux passages de 100ml chacun. La fraction organique est amenée à sec (soit 4,2g) ; elle contient outre le tensioactif, l'heptakis (6-désoxy-6-sulfonyl) cyclomalto-heptaose.

Ce résidu organique est additionné de 1ml de DMSO et d'un mélange organique contenant 250ml de méthanol, 150ml d'acétone et 1ml de solution aqueuse de soude 4N. La solution trouble obtenue est alors centrifugée à 5000 t/min pendant 20 min. Les culots récupérés sont dissous dans de l'eau distillée et la solution filtrée est lyophilisée.

Un spectre RMN du ¹³C montre la présence du seul sel de sodium de l'heptakis(6-désoxy-6-sulfonyl) cyclomalto-heptaose. On en obtient 700mg, ce qui correspond à un rendement de 85% par rapport à l'halogénure de départ.

Les caractéristiques de ce sel de sodium sont les suivantes :
- RMN du ¹³C (dand D₂O avec du DMSO pris comme référence à 39,6 ppm), δppm : C-1 102,5 ; C-4 83,34 ; C-2, C-3, C-5 74,10, 73,31, 69,08 ; C-6 51,64.
- [α]_{*D*} = (c, H₂O) + 76,
- point de fusion = + 265°C.

La microanalyse calculée pour C₄₂H₆₃O₄₉S₇Na₇ donne : C% 29,03 ; H% 3,36 ; S% 12,90 ; Na% 9, et

La microanalyse trouvée est : C% 29,00 , H% 3,80 ; S% 12,65 ; Na% 7,8.

### EXEMPLE 2 : Synthèse du sel de sodium de l'hexakis(6-désoxy-6-sulfonyl)cyclomalto-hexaose.

A 120ml de l'émulsion ci-dessus d'agent tensioactif, sont ajoutés 900mg (7,14mmol) de sulfite de sodium et 900mg (3,31 méquiv.) d'hexakis (6-désoxy-6-iodo) cyclomalto-hexaose, préparé comme dans le document (8). Le mélange est porté à 100°C pendant 24 h. Après refroidissement, le mélange réactionnel est extrait par du dichlorométhane en deux passages de 100ml chacun. La fraction organique est amenée à sec (soit 4,2g) ; elle contient outre le tensioactif, l'hexakis (6-désoxy-6-sulfonyl) cyclomalto-hexaose.

Ce résidu organique est additionné de DMSO et d'un mélange organique comme dans l'exemple 1. La solution trouble obtenue est alors centrifugée comme ci-dessus. Les culots récupérés sont dissous dans de l'eau distillée et la solution filtrée est lyophilisée.

Un spectre RMN du ¹³C montre la présence du seul sel de sodium de l'hexakis (6-désoxy-6-sulfonyl) cyclomalto-hexaose. On en obtient 653mg, ce qui correspond à un rendement de 80% par rapport à l'halogénure de départ.

Les caractéristiques de ce sel de sodium sont les suivantes :
- RMN du ¹³C (dand D₂O avec du DMSO pris comme référence à 39,6 ppm), δppm : C-1 102,5 ; C-4 83,34 ; C-2, C-3, C-5 74,10, 73,31, 69,08 ; C-6 51,64.
- [α]_{*D*} = (c, H₂O) + 75,
- Point de fusion = + 258°C.

La microanalyse calculée pour C₃₆H₅₄O₄₂S₆Na₆ donne : C% 29,03 ; H% 3,63 ; S% 12,90 ; Na% 9, et

La microanalyse trouvée est : C% 28,08 ; H% 3,9 ; S% 11,7 ; Na% 6,9.

### EXEMPLE 3 : Synthèse du sel de sodium de l'heptakis (6-sulfonyl) cyclomalto-heptaose.

Les 900mg d'heptakis (6-désoxy-6-iodo) cyclomalto-heptaose de l'exemple 3 sont remplacés par 750mg d'heptakis (6-bromo-6-désoxy) cyclomalto-heptaose.

Le produit obtenu possède les mêmes caractéristiques physiques que celui obtenu à partir du dérivé iodé correspondant de l'exemple 3.

Les sels de sodium de l'invention peuvent être utilisés comme support de substances actives et en particulier comme encapsulant afin de les transporter vers les sites cibles du corps humain. Ces substances actives sont en particulier des neurotropes utilisées pour le traitement des épilepsies, en gériatrie et contre les tumeurs. Ces sels de sodium peuvent aussi servir pour le transport d'anti-hypertenseurs et de diurétiques ainsi que de l'AZT.

Leur solubilité importante dans l'eau, leur stabilité et leur non destruction des parois des hématies (du fait de leurs groupements anioniques) permettent l'injection par voie veineuse de ces substances actives jusqu'alors insolubles dans l'eau.

## Revendications

1. Dérivé sulfoné de cyclomalto-oligosaccharide, caractérisé en ce qu'il présente la formule (I) suivante : 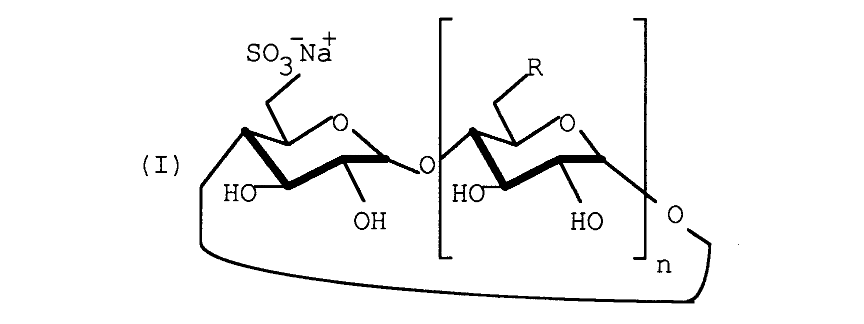 dans laquelle n est un entier allant de 2 à 50 et R représente -SO₃⁻Na⁺ ou -OH, R pouvant être différent d'un cycle à l'autre.

2. Dérivé selon la revendication 1, caractérisé en ce que n vaut de 2 à 10.

3. Dérivé selon la revendication 1 ou 2, caractérisé en ce que R=-SO₃⁻Na⁺ pour tous les cycles et n vaut 5 ou 6.

4. Procédé de préparation d'un dérivé sulfoné selon l'une quelconque des revendications 1 à 3, caractérisé en ce qu'on fait réagir du sulfite de sodium sur un halogénure d'un cyclomalto-oligosaccharide correspondant, en présence d'un agent de transfert de phase, cet halogénure comportant au moins un atome d'halogène en position 6 d'un cycle glucosique.

5. Procédé selon la revendication 4, caractérisé en ce que l'halogénure est un iodure ou un bromure.

6. Procédé selon la revendication 4 ou 5, caractérisé en ce que l'agent de transfert de phase a une tête polaire chargée positivement par un groupement ammonium et une partie hydrophobe à groupement alkyle chargé négativement par un contre-ion non compétiteur de l'anion sulfite.

7. Procédé selon la revendication 4, caractérisé en ce que l'agent de transfert de phase est le sulfate de méthyltrioctylammonium.

8. Procédé selon l'une quelconque des revendications 4 à 7, caractérisé en ce que la réaction du sulfite de sodium sur l'halogénure est effectué à un pH allant de 4 à 8.

9. Support de substances actives, caractérisé en ce qu'il contient un dérivé sulfoné selon l'une quelconque des revendications 1 à 3.

10. Support selon la revendication 9, caractérisé en ce qu'il se présente sous forme d'encapsulant.
